# EUROPEAN PATENT SPECIFICATION

(11) **EP 2 245 060 B1**
(45) Date of publication and mention of the grant of the patent: **01.11.2017**
(21) Application number: 09706431.5
(22) Date of filing: 29.01.2009
(51) Int. Cl.: C07K 14/81, C07K 14/415, C11D 3/386

(54) **LIQUID ENZYME COMPOSITION**
FLÜSSIGE ENZYMZUSAMMENSETZUNG
COMPOSITION ENZYMATIQUE LIQUIDE

(30) Priority: 01.02.2008 EP 08150977
(43) Date of publication of application: 03.11.2010
(73) Proprietor: Novozymes A/S, 2880 Bagsvaerd (DK)
(72) Inventor: SIMONSEN, Ole, 2860 Soeborg (DK); KNOETZEL, Juergen Carsten Franz, 2100 Copenhagen Oe (DK); NIELSEN, Lone Kierstein, 2800 Kongens Lyngby (DK)
(86) International application number: PCT/EP2009/050977
(87) International publication number: WO 2009/095425

(56) References cited:
- WO-A-92/05239
- WO-A-93/17086
- US-A1- 2004 038 845
- OHTSUBO K ET AL: "The amino acid sequence of a 20 kDa bifunctional subtilisin/alpha-amylase inhibitor from bran [correction of brain] of rice (Oryza sativa L.) seeds." FEBS LETTERS 31 AUG 1992, vol. 309, no. 1, 31 August 1992 (1992-08-31), pages 68-72, XP002475081 ISSN: 0014-5793
- YAMASAKI TERUYUKI ET AL: "Rice bifunctional alpha-amylase/subtilisin inhibitor: Cloning and characterization of the recombinant inhibitor expressed in Escherichia coli" BIOSCIENCE BIOTECHNOLOGY AND BIOCHEMISTRY, vol. 70, no. 5, May 2006 (2006-05), pages 1200-1209, XP002475080 ISSN: 0916-8451
- PEKKARINEN ANJA I ET AL: "Kinetics of the inhibition of Fusarium serine proteinases by barley (Hordeum vulgare L.) inhibitors" JOURNAL OF AGRICULTURAL AND FOOD CHEMISTRY, vol. 55, no. 7, April 2007 (2007-04), pages 2736-2742, XP002475082 ISSN: 0021-8561
- GVOZDEVA EKATERINA L ET AL: "Enzymatic oxidation of the bifunctional wheat inhibitor of subtilisin and endogenous alpha-amylase" FEBS (FEDERATION OF EUROPEAN BIOCHEMICAL SOCIETIES) LETTERS, vol. 334, no. 1, 1993, pages 72-74, XP002475083 ISSN: 0014-5793

### SEQUENCE LISTING

<110> Novozymes A/S
<120> Liquid enzyme composition
<130> 11026.204-WO
<160> 3
<170> PatentIn version 3.5
<210> 1
   <211> 200
   <212> PRT
   <213> Oryza sativa
<220>
   <221> mat_peptide
   <222> (23)..(200)
<400> 1
   Met Val Ser Leu Arg Leu Pro Leu Ile Leu Leu Ser Leu Leu Ala Ile
           -20                 -15                 -10
   Ser Phe Ser Cys Ser Ala Ala Pro Pro Pro Val Tyr Asp Thr Glu Gly
       -5              -1  1               5                   10
   His Glu Leu Ser Ala Asp Gly Ser Tyr Tyr Val Leu Pro Ala Ser Pro
                   15                  20                  25
   Gly His Gly Gly Gly Leu Thr Met Ala Pro Arg Val Leu Pro Cys Pro
               30                  35                  40
   Leu Leu Val Ala Gln Glu Thr Asp Glu Arg Arg Lys Gly Phe Pro Val
           45                  50                  55
   Arg Phe Thr Pro Trp Gly Gly Ala Ala Ala Pro Glu Asp Arg Thr Ile
       60                  65                  70
   Arg Val Ser Thr Asp Val Arg Ile Arg Phe Asn Ala Ala Thr Ile Cys
   75                  80                  85                  90
   Val Gln Ser Thr Glu Trp His Val Gly Asp Glu Pro Leu Thr Gly Ala
                   95                  100                 105
   Arg Arg Val Val Thr Gly Pro Leu Ile Gly Pro Ser Pro Ser Gly Arg
               110                 115                 120
   Glu Asn Ala Phe Arg Val Glu Lys Tyr Gly Gly Gly Tyr Lys Leu Val
           125                 130                 135
   Ser Cys Arg Asp Ser Cys Gln Asp Leu Gly Val Ser Arg Asp Gly Ala
       140                 145                 150
   Arg Ala Trp Leu Gly Ala Ser Gln Pro Pro His Val Val Val Phe Lys
   155                 160                 165                 170
   Lys Ala Arg Pro Ser Pro Pro Glu
                   175
<210> 2
   <211> 203
   <212> PRT
   <213> Hordeum vulgare
<220>
   <221> mat_peptide
   <222> (24)..(203)
<400> 2
   Met Gly Ser Arg Arg Ala Gly Ser Ser Ser Ser Pro Leu Phe Trp Pro
               -20                 -15                 -10
   Ala Pro Pro Ser Arg Ala Ala Asp Pro Pro Pro Val His Asp Thr Asp
           -5              -1  1               5
   Gly His Glu Leu Arg Ala Asp Ala Asn Tyr Tyr Val Leu Ser Ala Asn
   10                  15                  20                  25
   Arg Ala His Gly Gly Gly Leu Thr Met Ala Pro Gly His Gly Arg His
                   30                  35                  40
   Cys Pro Leu Phe Val Ser Gln Asp Pro Asn Gly Gln His Asp Gly Phe
               45                  50                  55
   Pro Val Arg Ile Thr Pro Tyr Gly Val Ala Pro Ser Asp Lys Ile Ile
           60                  65                  70
   Arg Leu Ser Thr Asp Val Arg Ile Ser Phe Arg Ala Tyr Thr Thr Cys
       75                  80                  85
   Leu Gln Ser Thr Glu Trp His Ile Asp Ser Glu Leu Ala Ala Gly Arg
   90                  95                  100                 105
   Arg His Val Ile Thr Gly Pro Val Lys Asp Pro Ser Pro Ser Gly Arg
                   110                 115                 120
   Glu Asn Ala Phe Arg Ile Glu Lys Tyr Ser Gly Ala Glu Val His Glu
               125                 130                 135
   Tyr Lys Leu Met Ser Cys Gly Asp Trp Cys Gln Asp Leu Gly Val Phe
           140                 145                 150
   Arg Asp Leu Lys Gly Gly Ala Trp Phe Leu Gly Ala Thr Glu Pro Tyr
       155                 160                 165
   His Val Val Val Phe Lys Lys Ala Pro Pro Ala
   170                 175                 180
<210> 3
   <211> 180
   <212> PRT
   <213> Triticum aestivum
<400> 3
   Asp Pro Pro Pro Val His Asp Thr Asp Gly Asn Glu Leu Arg Ala Asp
   1               5                   10                  15
   Ala Asn Tyr Tyr Val Leu Pro Ala Asn Arg Ala His Gly Gly Gly Leu
               20                  25                  30
   Thr Met Ala Pro Gly His Gly Arg Arg Cys Pro Leu Phe Val Ser Gln
           35                  40                  45
   Glu Ala Asp Gly Gln Arg Asp Gly Leu Pro Val Arg Ile Ala Pro His
       50                  55                  60
   Gly Gly Ala Pro Ser Asp Lys Ile Ile Arg Leu Ser Thr Asp Val Arg
   65                  70                  75                  80
   Ile Ser Phe Arg Ala Tyr Thr Thr Cys Val Gln Ser Thr Glu Trp His
                   85                  90                  95
   Ile Asp Ser Glu Leu Val Ser Gly Arg Arg His Val Ile Thr Gly Pro
               100                 105                 110
   Val Arg Asp Pro Ser Pro Ser Gly Arg Glu Asn Ala Phe Arg Ile Glu
           115                 120                 125
   Lys Tyr Ser Gly Ala Glu Val His Glu Tyr Lys Leu Met Ala Cys Gly
       130                 135                 140
   Asp Ser Cys Gln Asp Leu Gly Val Phe Arg Asp Leu Lys Gly Gly Ala
   145                 150                 155                 160
   Trp Phe Leu Gly Ala Thr Glu Pro Tyr His Val Val Val Phe Lys Lys
                   165                 170                 175
   Ala Pro Pro Ala
               180

## Description

### REFERENCE TO A SEQUENCE LISTING

This application contains a Sequence Listing in computer readable form. The computer readable form is incorporated herein by reference.

### FIELD OF THE INVENTION

The present invention relates to a liquid detergent composition comprising a surfactant, a subtilisin and a subtilisin inhibitor.

### BACKGROUND OF THE INVENTION

Proteases, especially serine proteases such as subtilisins, are widely used as ingredients in commercial detergents. A major problem in formulating protease-containing detergents, especially liquid detergents, is that of ensuring enzyme stability during storage.

WO 1992/003529 (Novo Nordisk) discloses a detergent composition comprising a protease and one or more other enzymes, as well as comprising a reversible protease inhibitor of the peptide or protein type, particularly an inhibitor of family VI.

WO 1992/005239 (Novo Nordisk) discloses a detergent composition and additive comprising a protease and a reversible protease inhibitor of the peptide or protein type, wherein the ratio of dissociation constant to the protease concentration in the range from 0.006 to 6. When the protease is subtilisin, the protease inhibitor is preferably a modified subtilisin inhibitor of Family VI.

BASI, RASI and WASI (barley, rice and wheat alpha-amylase/subtilisin inhibitor) are described by B.C. Bønsager et al., The Journal of Biological Chemistry, vol. 280, pp. 14855-14864 (2005); and T. Yamasaki et al., Biosci. Biotechnol. Biochem., 70 (5), 1200-1209 (2006).

### SUMMARY OF THE INVENTION

The inventors have found that incorporation of a subtilisin inhibitor such as RASI, BASI, WASI (bifunctional alpha-amylase/subtilisin inhibitors of rice, barley and wheat) into a liquid detergent which contains a subtilisin can stabilize the subtilisin and/or a second enzyme and can release the enzyme when the detergent composition is diluted.

Accordingly, the invention provides a liquid detergent composition comprising a surfactant, a subtilisin and a subtilisin inhibitor wherein the inhibitor has an amino acid sequence which has at least 90% identity to the mature peptide of SEQ ID NO: 1 (RASI), 2 (BASI) or 3 (WASI) or which comprises a sequence having at least 90% identity to residues 68-97 of SEQ ID NO: 1, 67-96 of SEQ ID NO: 2 or 67-96 of SEQ ID NO: 3; and wherein the inhibitor can stabilize the subtilisin and can release the enzyme when the detergent composition is diluted.

### DETAILED DESCRIPTION OF THE INVENTION

### Subtilisin inhibitor

The subtilisin inhibitor is a polypeptide which may have the amino acid sequence of RASI, BASI or WASI (SEQ ID NO: 1, 2 or 3), or a close homologue of any of these. Thus, the inhibitor has at least 90% identity to the mature peptide or it comprises a sequence having at least 90% identity to residues 68-97 of SEQ ID NO: 1 (RASI), 67-96 of SEQ ID NO: 2 (BASI) or 67-96 of SEQ ID NO: 3. The identity may particularly be at least 90%, at least 95% or at least 98%.

### Sequence identity

The degree of identity between two amino acid sequences is calculated as the number of exact matches in an alignment of the two sequences, divided by the length of the shorter of the two sequences. The result is expressed in percent identity. An exact match occurs when the two sequences have identical amino acid residues in the same positions of the overlap. The length of a sequence is the number of amino acid residues in the sequence.

The alignment of the two amino acid sequences may be determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

Alternatively, the alignment may be done by using the MegAlign program (version 7) developed by DNASTAR Inc., part of the Lasergene suite, based on Hein, J.J. (1990). "Unified approach to alignment and phylogenies." In Methods in Enzymology, Vol. 183: pp. 626-645. Using the Jotun Hein Method and the settings GAP PENALTY = 11, GAP LENGTH PENALTY = 3 for multiple alignments and KTUPLE = 2 for pairwise alignments a series of percentage identity values can be calculated.

### Subtilisin

The subtilisin may be of animal, vegetable or microbial origin, preferably an alkaline microbial protease. Examples of subtilisins are especially those derived from *Bacillus,* e.g., subtilisin Novo, subtilisin Carlsberg, subtilisin BPN', subtilisin 309, subtilisin 147 and subtilisin 168 (described in WO 89/06279).

Examples of commercially available subtilisins (peptidases) include Kannase™, Everlase™, Esperase™, Alcalase™, Neutrase™, Durazym™, Savinase™, Ovozyme™, Liquanase™, Polarzyme™, Pyrase™, Bio-Feed™ Pro and Clear-Lens™ Pro (all available from Novozymes A/S, Bagsvaerd, Denmark). Preferred subtilisins include those described in WO 1998/020115, WO 01/44452, WO 01/58275, WO 01/58276, WO 2003/006602, and WO 2004/099401.

Other commercially available subtilisins include Ronozyme™ Pro, Maxatase™, Maxacal™, Maxapem™, Opticlean™, Properase™, Purafect™, Purafect Ox™ and Purafact Prime™ (available from Genencor International Inc., Gist-Brocades, BASF, or DSM Nutritional Products).

### Optional second enzyme

In addition to the subtilisin, the liquid composition may optionally comprise one or more other enzymes, e.g. selected among amylases, lipolytic enzymes (particularly lipases), cellulases, mannanases and oxidoreductases.

The amylase may be an alpha-amylase of bacterial or fungal origin, e.g. an alpha-amylase from *B. licheniformis,* described in GB 1,296,839. Commercially available amylases are Duramyl™, Termamyl™, Stainzyme™, Stainzyme Plus™, Termamyl Ultra™, Fungamyl™ and BAN™ (available from Novozymes A/S) and Rapidase™, Maxamyl P™, Purastar and Purastar OxAm (available from Gist-Brocades and Genencor Inc.).The cellulase may be of bacterial or fungal origin. It may be a fungal cellulase from *Humicola insolens* (US 4,435,307) or from *Trichoderma,* e.g. *T. reesei* or *T. viride.* Examples of cellulases are described in EP 0 495 257. Commercially available cellulases include Carezyme™, Celluzyme™, Endolase™ (available from Novozymes), Puradax, Puradax HA, and Puradax EG (available from Genencor).

The oxidoreductase may be a peroxidase or an oxidase such as a laccase. The peroxidase may be of plant, bacterial or fungal origin. Examples are peroxidases derived from a strain of *Coprinus,* e.g., *C*. *cinerius* or *C*. *macrorhizus,* or from a strain of *Bacillus,* e.g., *B. pumilus,* particularly peroxidase according to WO 91/05858. Suitable laccases herein include those of bacterial or fungal origin. Examples are laccases from *Trametes,* e.g., *T. villosa* or *T. versicolor,* or from a strain of *Coprinus,* e.g., *C*. *cinereus,* or from a strain of *Myceliophthora,* e.g., *M*. *thermophila.*

The lipolytic enzyme may be a lipase or cutinase of bacterial or fungal origin. Examples include a lipase from *Thermomyces lanuginosus* (*Humicola lanuginosa)* described in EP 258 068 and EP 305 216, a *Rhizomucor miehei* lipase, e.g., as described in EP 238 023, a *Candida* lipase, such as a *C*. *antarctica* lipase, e.g., the *C*. *antarctica* lipase A or B described in EP 214 761, a *Fusarium oxysporum* lipase (WO 98/26057), a *Pseudomonas* lipase such as a *P*. *pseudoalcaligenes* and *P. alcaligenes* lipase, e.g., as described in EP 218 272, a *P. cepacia* lipase, e.g., as described in EP 331 376, a *P. stutzeri* lipase, e.g., as disclosed in BP 1,372,034, a *P. fluorescens* lipase, a *Bacillus* lipase, e.g., a *B. subtilis* lipase (Dartois et al., (1993), Biochemica et Biophysica acta 1131, 253-260), a *B. stearothermophilus* lipase (JP 64/744992), *B. pumilus* lipase (WO 91/16422), *Penicillium camenbertii* lipase (Yamaguchi et al., (1991), Gene 103, 61-67), the *Geotrichum candidum* lipase (Shimada, Y. et al., (1989), J. Biochem. 106, 383-388), and various *Rhizopus* lipases such as a *R. delemar* lipase (Hass, M.J et al., (1991), Gene 109, 117-113), a *R. niveus* lipase (Kugimiya et al., (1992), Biosci. Biotech. Bio-chem. 56, 716-719) and a *R. oryzae* lipase. Additional examples are cutinase from *Pseudomonas mendocina* (WO 88/09367), cutinase from *Fusarium solani pisi* (WO 90/09446) and cutinase from *Humicola insolens* (WO 2001/092502). The lipolytic enzyme may be a lipase variant, e.g. described in WO 2000/060063.

Examples of commercially available lipases include Lipex™, Lipoprime™, Lipopan™, Lipopan F™, Lipopan Xtra™, Lipolase™, Lipolase™ Ultra, Lipozyme™, Palatase™, Resinase™, Novozym™ 435 and Lecitase™ (all available from Novozymes A/S). Other commercially available lipases include Lumafast™ (*Pseudomonas mendocina* lipase from Genencor International Inc.); Lipomax™ (*Ps. pseudoalcaligenes* lipase from Gist- Brocades/Genencor Int. Inc.; and *Bacillus* sp. lipase from Solvay enzymes. Further lipases are available from other suppliers such as Lipase P "Amano" (Amano Pharmaceutical Co. Ltd.).

Examples of mannanases include Mannaway™ (product of Novozymes) and MannaStar (product of Genencor).

### Liquid detergent composition

The invention is particularly applicable to the formulation of liquid detergents where enzyme stability problems are pronounced. The liquid detergent may be aqueous, typically containing 20-70% water and 0-20% organic solvent (hereinafter, percentages by weight).

The detergent comprises a surfactant which may be anionic, non-ionic, cationic, amphoteric or a mixture of these types. The detergent will usually contain 5-30% anionic surfactant such as linear alkyl benzene sulphonate (LAS), alpha-olefin sulphonate (AOS), alcohol ethoxy sulphate (AES) or soap. It may also contain 3-20% anionic surfactant such as nonyl phenol ethoxylate or alcohol ethoxylate.

The pH (measured in aqueous detergent solution) will usually be neutral or alkaline, e.g. 7-10. The detergent may contain 1-40% of a detergent builder such as zeolite, phosphate, phosphonate, citrate, NTA, EDTA or DTPA, or it may be unbuilt (i.e. essentially free of a detergent builder). It may also contain other conventional detergent ingredients, e.g. fabric conditioners, foam boosters, bactericides, optical brighteners and perfumes.

The detergent composition may be a fabric cleaning compositions, hard surface cleansing compositions, light duty cleaning compositions including dish cleansing compositions and automatic dishwasher detergent compositions.

The liquid composition may comprise from about 0.0001 % to about 10%, more particularly from about 0.001% to about 1%, and most particularly from about 0.01% to about 0.1% of the inhibitor.

The molar ratio of the inhibitor to the serine protease may be from about 100:1 to about 1:1, more particularly from about 10:1 to about 1.5:1, and most particularly about 3:1.

Thus, a stabilized liquid enzyme formulation typically contains 1-10% by weight of enzyme protein (total of serine protease and optional second enzyme) and 2-25% by weight of the inhibitor

A liquid detergent formulation will typically contain 0.04-40 micromolar enzyme or 1-1000 mg/l of pure enzyme protein and about 3 times more of the inhibitor, i.e. 0.12-120 micromolar of inhibitor.

The liquid detergent composition may contain water and other solvents as carriers. Low molecular weight primary or secondary alcohols exemplified by methanol, ethanol, propanol, and iso-propanol are suitable. Monohydric alcohols are preferred for solubilizing surfactants, but polyols such as those containing from about 2 to about 6 carbon atoms and from about 2 to about 6 hydroxy groups (*e*.*g*., 1,3-propanediol, ethylene glycol, glycerine, and 1,2-propanediol) can also be used. The compositions may contain from about 5% to about 90%, typically from about 10% to about 50% of such carriers.

The detergent compositions herein will preferably be formulated such that during use in aqueous cleaning operations, the wash water will have a pH between about 6.8 and about 11. Finished products are typically formulated at this range. Techniques for controlling pH at recommended usage levels include the use of, for example, buffers, alkalis, and acids. Such techniques are well known to those skilled in the art.

When formulating the hard surface cleaning compositions and fabric cleaning compositions of the present invention, the formulator may wish to employ various builders at levels from about 5% to about 50% by weight. Typical builders include the 1-10 micron zeolites, polycarboxylates such as citrate and oxydisuccinates, layered silicates, phosphates, and the like. Other conventional builders are listed in standard formularies.

### EXAMPLES

### Example 1: Stabilization of Savinase by BASI in 4 different detergents

Four liquid detergents were prepared with the compositions shown below:
Detergent 1 (American liquid-type detergent without LAS) Dosage in Assay:1.5g/L.
Detergent 2 (American liquid-type detergent with LAS) Dosage in Assay:1.5g/L.
Detergent 3 (European liquid-type detergent without LAS) Dosage in Assay: 6g/L.
Detergent 4 (European liquid-type detergent with LAS) Dosage in Assay: 6g/L.

| Component, % w/w | Det 1 | Det 2 | Det 3 | Det 4 |
|---|---|---|---|---|
| Sodium alkylethoxy sulphate (C9-15, 2EO) | 14.0 | 7.4 | | |
| Sodium dodecyl benzene sulphonate (LAS) | | 5.5 | | 10.0 |
| Sodium lauryl sulphate | | | 17.0 | |
| Sodium toluene sulphonate | 3.0 | 1.0 | 3.0 | 1.0 |
| Sodium xylene sulphonate | | | | 4.4 |
| Oleic acid | 4.0 | | 10.0 | 13.0 |
| Primary alcohol ethoxylate (C12-15, 7EO) | 2.5 | 3.0 | 5.0 | 7.0 |
| Primary alcohol ethoxylate (C12-15, 3EO) | 2.0 | 2.5 | 4.0 | 6.0 |
| Ethanol | 2.1 | 1.0 | 3.0 | 4.0 |
| Sodium carbonate | 4.5 | 4.0 | 0.5 | |
| Tri-sodium citrate 2H₂O | 5.0 | 2.0 | 4.5 | 1.0 |
| pH (adjusted with NaOH) | 8.0 | 9.0 | 9.0 | 9.0 |
| De-ionized water: ad 100% | | | | |

An inhibitor assay was conducted as follows:
100 micro-L Savinase (1 micro-g/ml, 37 nM) and 100 micro-L buffer (50mM Glycine, 150mM KCl, 0.05mM CaCl₂, 0.01Triton X-100, pH 9.8) was incubated for 30 min at room temperature in the absence or presence of detergents 1, 2, 3 or 4 (minus inhibitor). 100 micro-L Savinase (1µg/ml, 37 nM) and 100 micro-L BASI (13,8 micro-g/ml, 690 nM) was incubated for 30 min at room temperature in the absence or presence of detergents 1, 2, 3 or 4 (plus inhibitor).

Subsequently, 50 micro-L dissolved 4.8 mM serine protease substrate Suc-Ala-Ala-Pro-Phe-pNA (Sigma S-7388) in 50 mM Glycine, pH 9.8 was added, and the activity was measured at 405 nm over 7 min. Results are expressed as residual activity compared to the Savinase activity in the absence of inhibitor in detergent 1, 2, 3, or 4.

| | Without inhibitor | With inhibitor | Residual activity |
|---|---|---|---|
| No detergent | 339 | 42 | 12% |
| Detergent 1 | 324 | 40 | 12% |
| Detergent 2 | 290 | 44 | 15% |
| Detergent 3 | 322 | 42 | 13% |
| Detergent 4 | 281 | 44 | 16% |

The results show that BASI inhibits Savinase efficiently in the absence of detergents and equally well in the presence of detergents 1, 2, 3, and 4. Thus, the inhibition efficiency of BASI is not influenced by the presence of LAS in detergents 2 and 4 as can be seen from the almost identical results for the respective detergents 1 and 3 without LAS.

### Example 2: Stabilization of serine protease and lipase

Two liquid detergents were prepared as follows:

| Component | Reference detergent | BASI detergent (0.044% BASI) |
|---|---|---|
| Detergent base 3 (Example 1) | 22.0 g | 22.0 g |
| Serine protease (Savinase 16.0 LEX) | 0.12 g | 0.12 g |
| Lipase (Lipex 100L) | 0.12 g | 0.12 g |
| De-ionized water | 2.9 g | |
| BASI-solution (2.56 mg/ml) | | 4.6 g |

The detergents were placed in closed glasses at 30°C and 35°C. Residual activity of lipase and serine protease was measured (by comparison to a reference stored at -18°C) at different times (serine protease measured by hydrolysis of N,N-dimethylcasein at 40°C, pH 8.3 and lipase measured by hydrolysis of p-nitrophenyl valerate at 40°C, pH 7.7).

| % residual activity | Residual serine protease activity | | | Residual lipase activity | |
|---|---|---|---|---|---|
| Detergent | 3 days 30°C | 1 week 35°C | 2 weeks 35°C | 3 days 30°C | 1 week 35°C |
| Reference | 63 | 2.3 | 0.4 | 25 | 3.3 |
| 0.044% BASI | 96 | 78 | 48 | 86 | 31 |

From the table it is clearly seen that BASI significantly stabilizes both enzymes

## Claims

1. A liquid detergent composition comprising a surfactant, a subtilisin and a subtilisin inhibitor wherein the inhibitor has an amino acid sequence which has at least 90% identity to the mature peptide of SEQ ID NO: 1 (RASI), 2 (BASI) or 3 (WASI) or which comprises a sequence having at least 90% identity to residues 68-97 of SEQ ID NO: 1, 67-96 of SEQ ID NO: 2 or 67-96 of SEQ ID NO: 3; and wherein the inhibitor can stabilize the subtilisin and can release the enzyme when the detergent composition is diluted.

2. The composition of claim 1, which further comprises a second enzyme.

3. The composition of claim 1 or 2, wherein the second enzyme is selected from amylases, lipases, cellulases, mannanases and oxidoreductases.

4. The composition of any preceding claim, wherein the subtilisin and the inhibitor are present in a molar ratio of 1:100 to 1:1.

5. The composition of any preceding claim, wherein the inhibitor is present at a concentration of 0.0001 % to 10 % by weight.

## Patentansprüche

1. Flüssigwaschmittelzusammensetzung, die ein oberflächenaktives Mittel, ein Subtilisin und einen Subtilisininhibitor umfasst, wobei der Inhibitor eine Aminosäuresequenz aufweist, die mindestens 90% Identität zum reifen Peptid von SEQ ID NO: 1 (RASI), 2 (BASI) oder 3 (WASI) aufweist, oder die eine Sequenz mit mindestens 90% Identität zu Resten 68-97 von SEQ ID NO: 1, 67-96 von SEQ ID NO: 2 oder 67-96 von SEQ ID NO: 3 umfasst; und wobei der Inhibitor das Subtilisin stabilisieren kann und das Enzym freisetzen kann, wenn die Detergenszusammensetzung verdünnt ist.

2. Zusammensetzung nach Anspruch 1, die weiterhin ein zweites Enzym umfasst.

3. Zusammensetzung nach Anspruch 1 oder 2, wobei das zweite Enzym aus Amylasen, Lipasen, Cellulasen, Mannanasen und Oxidoreduktasen ausgewählt ist.

4. Zusammensetzung nach einem beliebigen voranstehenden Anspruch, wobei das Subtilisin und der Inhibitor in einem Molverhältnis von 1:100 bis 1:1 vorhanden sind.

5. Zusammensetzung nach einem beliebigen voranstehenden Anspruch, wobei der Inhibitor in einer Konzentration von 0,0001 % bis 10 % bezogen auf das Gewicht vorhanden ist.

## Revendications

1. Composition détergente liquide comprenant un tensioactif, une subtilisine et un inhibiteur de subtilisine dans laquelle l'inhibiteur a une séquence d'acides aminés qui présente une identité d'au moins 90 % avec le peptide mature de SEQ ID No: 1 (RASI), 2 (BASI) ou 3 (WASI) ou qui comprend une séquence présentant une identité d'au moins 90 % avec les résidus 68-97 de SEQ ID No: 1, 67-96 de SEQ ID No: 2 ou 67-96 de SEQ ID No: 3, et dans laquelle l'inhibiteur peut stabiliser la subtilisine et peut libérer enzyme quand la composition détergente est diluée.

2. Composition selon la revendication 1, qui comprend en outre une seconde enzyme.

3. Composition selon la revendication 1 ou 2, dans laquelle la seconde enzyme est choisie parmi les amylases, les lipases, les cellulases, les mannanases et les oxydoréductases.

4. Composition selon l'une quelconque des revendications précédentes, dans laquelle la subtilisine et l'inhibiteur sont présents en un rapport molaire de 1:100 à 1:1.

5. Composition selon l'une quelconque des revendications précédentes, dans laquelle l'inhibiteur est présent à une concentration de 0,0001 à 10 % en poids.
